# EUROPEAN PATENT SPECIFICATION

(11) **EP 2 600 835 B1**
(45) Date of publication and mention of the grant of the patent: **30.05.2018**
(21) Application number: 11815206.5
(22) Date of filing: 02.08.2011
(51) Int. Cl.: A61K 31/4725, A61K 31/4178, A61K 31/55, A61P 31/12, A61P 31/14

(54) **COMBINATIONS OF HEPATITIS C VIRUS INHIBITORS**
KOMBINATIONEN VON HEMMERN DES HEPATITIS-C-VIRUS
COMBINAISONS D'INHIBITEURS DU VIRUS DE L'HÉPATITE C

(30) Priority: 06.08.2010 US 371399 P
(43) Date of publication of application: 12.06.2013
(73) Proprietor: Bristol-Myers Squibb Holdings Ireland, 6312 Steinhausen (CH)
(72) Inventor: GAO, Min, Wallingford Connecticut 06492 (US); GARDINER, David F., Pennington New Jersey 08534 (US); LEMM, Julie A., Wallingford Connecticut 06492 (US); MCPHEE, Fiona, Wallingford Connecticut 06492 (US); VOSS, Stacey A., Wallingford Connecticut 06492 (US)
(74) Representative: Reitstötter Kinzebach
(86) International application number: PCT/US2011/046285
(87) International publication number: WO 2012/018829

(56) References cited:
- WO-A1-2011/046811
- US-A1- 2004 106 559
- US-A1- 2004 106 559
- US-A1- 2007 270 405
- US-A1- 2007 270 405
- US-A1- 2008 050 336
- US-A1- 2008 050 336
- KRONENBERGER BERND ET AL: "Current and future treatment options for HCV.", ANNALS OF HEPATOLOGY 2009 APR-JUN, vol. 8, no. 2, April 2009 (2009-04), pages 103-112, XP002714684, ISSN: 1665-2681
- ASSELAH TARIK ET AL: "Protease and polymerase inhibitors for the treatment of hepatitis C.", LIVER INTERNATIONAL : OFFICIAL JOURNAL OF THE INTERNATIONAL ASSOCIATION FOR THE STUDY OF THE LIVER JAN 2009, vol. 29 Suppl 1, January 2009 (2009-01), pages 57-67, XP002714685, ISSN: 1478-3231
- L. A. PELOSI ET AL: "Effect on Hepatitis C Virus Replication of Combinations of Direct-Acting Antivirals, Including NS5A Inhibitor Daclatasvir", ANTIMICROBIAL AGENTS AND CHEMOTHERAPY, vol. 56, no. 10, 30 July 2012 (2012-07-30) , pages 5230-5239, XP055156471, ISSN: 0066-4804, DOI: 10.1128/AAC.01209-12

## Description

The present disclosure is generally directed to antiviral compounds and, more specifically, directed to combinations of compounds which can inhibit hepatitis C virus (HCV), compositions comprising such compounds, and their use in treating hepatitis C using such combinations.

HCV is a major human pathogen, infecting an estimated 170 million persons worldwide - roughly five times the number infected by human immunodeficiency virus type 1. A substantial fraction of these HCV-infected individuals develop serious progressive liver disease, including cirrhosis and hepatocellular carcinoma.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family are enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome due to the high error rate of the encoded RNA-dependent RNA polymerase which lacks a proof-reading capability. At least six major genotypes have been characterized, and more than 50 subtypes have been described with distribution worldwide. The clinical significance of the genetic heterogeneity of HCV has demonstrated a propensity for mutations to arise during monotherapy treatment; thus, additional treatment options for use are desired.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to herein as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions by both acting as a cofactor for the NS3 protease and assisting in the membrane localization of NS3 and other viral replicase components. The formation of a NS3/NS4A complex is necessary for proper protease activity resulting in increased proteolytic efficiency of the cleavage events. The NS3 protein also exhibits nucleoside triphosphatase and RNA helicase activities. NS4B is an integral membrane protein involved in formation of the membranous web where HCV replication complexes are thought to assemble. NS5B (also referred to herein as HCV polymerase) is a RNA-dependent RNA polymerase that is involved in the replication of HCV with other HCV proteins, including NS5A, in a replicase complex.

The current standard of care for treatment of most patients with chronic HCV infection is a regimen of pegylated interferon-alpha and ribavirin. However, a high proportion of patients fail to respond to this therapy and treatment is associated with significant side effects. Thus, there is a great need to develop safer and more effective therapies. Although a number of small molecule HCV inhibitors are currently in clinical trials, based on clinical data from several studies it is evident that combinations of inhibitors may be required to effect sustained viral response in HCV infected patients. Resistance emergence in patients during treatment, and post-treatment viral rebound, have been observed upon treatment with protease inhibitors, as well as, nucleoside and non-nucleoside HCV inhibitors. To achieve maximal efficacy and to potentially eradicate the virus, it will be critical to utilize combination therapies, especially those targeting distinct HCV viral targets. *In vitro* replicon-based combination studies have shown that additive to synergistic effects can be achieved with various combinations of HCV inhibitors.

Commonly owned patent application WO2008/021927 discloses compounds which inhibit the function of the NS5A protein encoded by HCV. U.S. Patent Ser. No. 6,995,174 discloses compounds which inhibit the function of the NS3 protease encoded by HCV. U.S. Patent Ser. No. 7,456,166 discloses compounds which inhibit the function of the NS5B polymerase. US20080050336, US20040106559 and US20070270405 disclose the presently claimed compounds of formula (I), (II) and (III) for use in treating HCV infections. The present disclosure teaches combinations of a specific HCV NS5A inhibitor, a specific HCV NS3 protease inhibitor, and NS5B polymerase inhibitors that are useful for the treatment of HCV.

In a first aspect the present disclosure provides a composition comprising a therapeutically effective amount of a compound of formula (I) 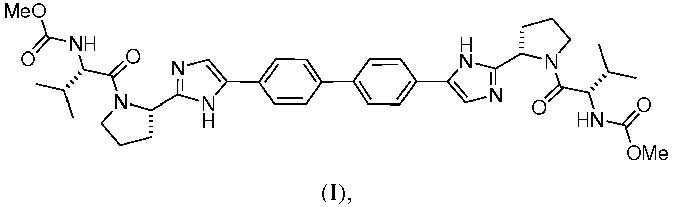 or a pharmaceutically acceptable salt thereof; a therapeutically effective amount of a compound of formula (II), 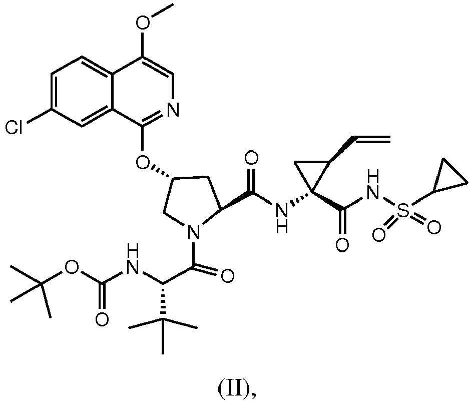 or a pharmaceutically acceptable salt thereof; a therapeutically effective amount of a compound effective to inhibit the function of HCV polymerase, i.e. the compound of formula (III) 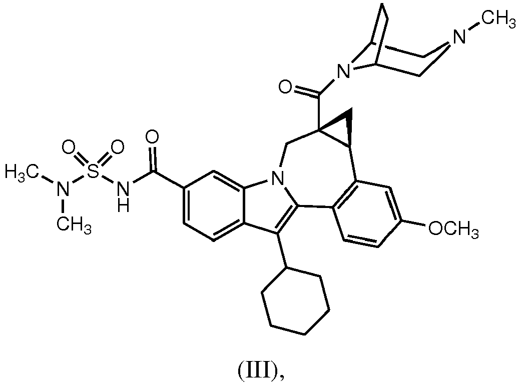 or a pharmaceutically acceptable salt thereof; and a pharmaceutically acceptable carrier.

In a first embodiment of the first aspect the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:20:5.

In a second embodiment of the first aspect the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:250:1000.

In a second aspect the present disclosure provides a therapeutically effective amount of a composition comprising a compound of formula (I) 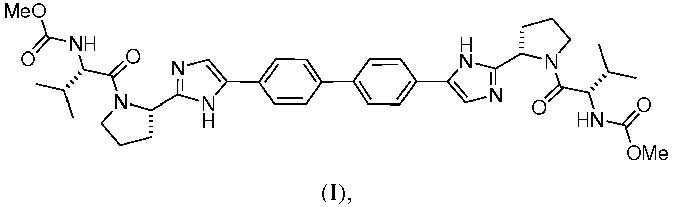 or a pharmaceutically acceptable salt thereof; a therapeutically effective amount of a compound of formula (II), 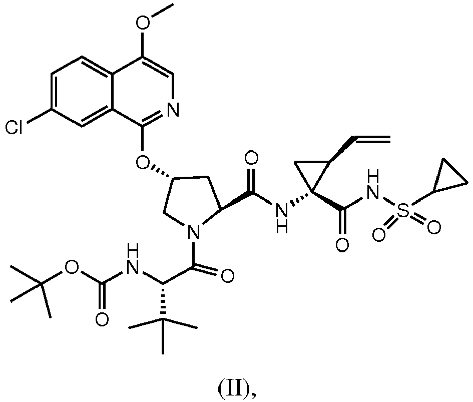 or a pharmaceutically acceptable salt thereof; a therapeutically effective amount of a compound effective to inhibit the function of HCV polymerase, i.e. the compound of formula (III) 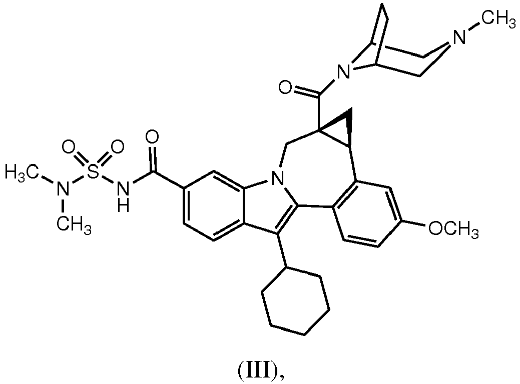 or a pharmaceutically acceptable salt thereof; and a pharmaceutically acceptable carrier, for use in treating HCV infection in a patient.

In a first embodiment of the second aspect the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:20:5.

In a second embodiment of the second aspect the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:250:1000.

As used in the present specification, the following terms have the meanings indicated:
As used herein, the singular forms "a", "an", and "the" include plural reference unless the context clearly dictates otherwise.

Certain compounds of the present disclosure may exist in different stable conformational forms which may be separable. Torsional asymmetry due to restricted rotation about an asymmetric single bond, for example because of steric hindrance or ring strain, may permit separation of different conformers, commonly known as atropisomers. The present disclosure includes each conformational isomer of these compounds and mixtures thereof.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are suitable for use in contact with the tissues of patients without excessive toxicity, irritation, allergic response, or other problem or complication commensurate with a reasonable benefit/risk ratio, and are effective for their intended use. The salts can be prepared during the final isolation and purification of the compounds or separately by reacting a suitable nitrogen atom with a suitable acid. Representative acid addition salts include acetate, adipate, alginate, citrate, aspartate, benzoate, benzenesulfonate, bisulfate, butyrate, camphorate, camphorsulfonate; digluconate, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting an acidic proton with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, and N,N'-dibenzylethylenediamine. Other representative organic amines useful for the formation of base addition salts include ethylenediamine, ethanolamine, diethanolamine, piperidine, and piperazine.

The disclosure provides pharmaceutical compositions, which are comprised of therapeutically effective amounts of compounds of Formulae (I) and (II), or pharmaceutically acceptable salts thereof, an NS5B inhibitor, i.e. the compound of Formula (III) and one or more pharmaceutically acceptable carriers, diluents, or excipients. The term "therapeutically effective amount," as used herein, refers to the total amount of each active component that is sufficient to show a meaningful patient benefit, e.g., a sustained reduction in viral load. When applied to an individual active ingredient, administered alone, the term refers to that ingredient alone. When applied to a combination, the term refers to combined amounts of the active ingredients that result in the therapeutic effect, whether administered in combination, serially, or simultaneously. The carrier(s), diluent(s), or excipient(s) must be acceptable in the sense of being compatible with the other ingredients of the formulation and not deleterious to the recipient thereof. In accordance with another aspect of the present disclosure there is also provided a process for the preparation of a pharmaceutical formulation including admixing compounds of Formulae (I) and (II), or pharmaceutically acceptable salts thereof, with an NS5B polymerase inhibitor, i.e. the compound of Formula (III), and one or more pharmaceutically acceptable carriers, diluents, or excipients. The term "pharmaceutically acceptable," as used herein, refers to those compounds, materials, compositions, and/or dosage forms which are suitable for use in contact with the tissues of patients without excessive toxicity, irritation, allergic response, or other problem or complication commensurate with a reasonable benefit/risk ratio, and are effective for their intended use.

Pharmaceutical formulations may be presented in unit dose forms containing a predetermined amount of active ingredients per unit dose. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Generally, treatment is initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

As the compositions of this disclosure comprise a combination of three or more compounds having anti-HCV activity, all compounds can be present in a dose that is less than or equal to the dosage normally administered in a monotherapy regimen. Dosage levels of between about 0.01 and about 250 milligram per kilogram ("mg/kg") body weight per day, preferably between about 0.05 and about 100 mg/kg body weight per day of each of the compounds of the present disclosure are typical in a monotherapy for the prevention and treatment of HCV mediated disease. The compositions of this disclosure may be co-formulated with one or more additional therapeutic or prophylactic agents, for example, in the form of a monolithic and/or bi/multi-layer tablet or may be administered separately from the therapeutic or prophylactic agent(s).

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, and water. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, and polyethylene glycol. Lubricants used in these dosage forms include sodium oleate, sodium chloride, and the like. Disintegrators include, without limitation, starch, methyl cellulose, agar, betonite, and xanthum gum. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of formulae (I) and (II), and pharmaceutically acceptable salts thereof, along with the NS5B polymerase inhibitor of formula (III) can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of formula (I) and (II), and pharmaceutically acceptable salts thereoof, along with the NS5B polymerase inhibitor of formula (III) may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols, or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles, and mouth washes.

Pharmaceutical formulations adapted for rectal administration may be presented as suppositories or as enemas.

Pharmaceutical formulations adapted for nasal administration wherein the carrier is a solid include a course powder which is administered in the manner in which snuff is taken, i.e., by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

The term "patient" includes both human and other mammals.

The term "treating" refers to: (i) preventing a disease, disorder or condition from occurring in a patient that may be predisposed to the disease, disorder, and/or condition but has not yet been diagnosed as having it; (ii) inhibiting the disease, disorder, or condition, i.e., arresting its development; and (iii) relieving the disease, disorder, or condition, i.e., causing regression of the disease, disorder, and/or condition.

Table 1 below lists some illustrative examples of compounds that can be administered with the compositions of this disclosure. The compositions of the disclosure can be administered with other anti-HCV activity compounds in combination therapy, either jointly or separately, or by combining the compounds into a composition.

**Reference Table 1**

| *Brand Name* | *Physiological Class* | *Type of Inhibitor or Target* | *Source Company* |
|---|---|---|---|
| HCV-796 | Antiviral | NS5B replicase inhibitor | Wyeth / Viropharma |
| NM-283 | Antiviral | NS5B replicase inhibitor | Idenix / Novartis |
| GL-59728 | Antiviral | NS5B replicase inhibitor | Gene Labs / Novartis |
| GL-60667 | Antiviral | NS5B replicase inhibitor | Gene Labs / Novartis |
| 2'C MeA | Antiviral | NS5B replicase inhibitor | Gilead |
| PSI-6130 | Antiviral | NS5B replicase inhibitor | Roche |
| R-1626 | Antiviral | NS5B replicase inhibitor | Roche |
| GL-59728 | Antiviral | NS5B replicase inhibitor | Genelabs |
| PF-00868554 | Antiviral | Non-nucleoside NS5B polymerase inhibitor | Pfizer |
| ANA-598 | Antiviral | Non-nucleoside NS5B polymerase inhibitor | Anadys Pharmaceuticals, Inc., San Diego, CA, USA |
| IDX-375 | Antiviral | Non-Nucleoside replicase inhibitor | Idenix Pharmaceuticals, Cambridge, MA, USA |
| IDX-184 | Antiviral | Nucleoside NS5B polymerase inhibitor | Idenix Pharmaceuticals, Cambridge, MA, USA |
| INX-189 | Antiviral | Prodrug of a nucleoside NS5B polymerase inhibitor | Inhibitex Inc, Alpharetta, GA, USA |
| BILB-1941 | Antiviral | Non-Nucleoside NS5B polymerase inhibitor | Boehringer Ingelheim Canada Ltd R&D, Laval, QC, Canada |
| BI-207127 | Antiviral | Non-Nucleoside NS5B polymerase inhibitor | Boehringer Ingelheim Canada Ltd R&D, Laval, QC, Canada |
| PSI-7851 | Antiviral | Nucleoside polymerase inhibitor | Pharmasset, Princeton, NJ, USA |
| PSI-938 | Antiviral | Nucleoside polymerase inhibitor | Pharmasset, Princeton, NJ, USA |
| PSI-879 | Antiviral | Nucleoside polymerase inhibitor | Pharmasset, Princeton, NJ, USA |
| VCH-759 | Antiviral | Non-nucleoside NS5B polymerase inhibitor | Vertex |
| VCH-916 | Antiviral | Non-nucleoside NS5B polymerase inhibitor | Vertex |
| VCH-222 | Antiviral | Non-nucleoside NS5B polymerase inhibitor | Vertex |
| BMS-929075 | Antiviral | Non-nucleoside NS5B polymerase inhibitor | Bristol-Myers Squibb |
| GS-9190 | Antiviral | Non-nucleoside NS5B polymerase inhibitor | Gilead |
| ABT-333 | Antiviral | Non-nucleoside NS5B polymerase inhibitor | Abbott Laboratories |
| ABT-072 | Antiviral | Non-nucleoside NS5B polymerase inhibitor | Abbott Laboratories |
| MK-0608 | Antiviral | Nucleoside polymerase inhibitor | Merck |
| MK-3281 | Antiviral | Non-nucleoside NS5B polymerase inhibitor | Merck |
| GSK-625433 | Antiviral | Non-nucleoside NS5B polymerase inhibitor | GSK |
| XTL-2125 | Antiviral | Non-nucleoside NS5B polymerase inhibitor | Presidio |

The compositions of the present disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

### EXAMPLES

### Compounds

The HCV NS5A inhibitor (compound of formula (I)) can be prepared following the procedure described in commonly owned patent applications WO2008/021927 or WO2009/020825. The HCV NS3 protease inhibitor (compound of formula (II)) can be prepared following the procedure described in commonly owned U.S. Patent Serial No. 6,995,174 and commonly owned U.S. patent application 12/547,158. The HCV NS5B inhibitor of formula (III) can be prepared by the procedure described in commonly owned U.S. Patent Serial No. 7,456,166.

The antiviral activity of the NS5A inhibitor (formula (I); 60 mg QD) and the NS3 protease inhibitor (formula (II); 600 mg BID) alone (Group A) or with pegylated interferon alpha and ribavirin (Group B) for 24 weeks in HCV genotype 1 null responders. The primary aim was to determine the proportion of subjects achieving undetectable HCV RNA (<10 IU/mL) at Weeks 2 and 4 of therapy and 24 weeks post-treatment. A Week 12 interim analysis was performed. Twenty-one patients (11 Group A, 10 Group B) were randomized in a sentinel cohort. The median age was 55 years; 13 were male and 16 were white.

**Table 2**

| | *Group A: NS5A Inhibitor and NS3 Protease Inhibitor (n*=*11)* | *Group B: NS5A Inhibitor, NS3 Protease Inhibitor*, *Pegylated Interferon Alpha and Ribarvirin (n*=*10)* |
|---|---|---|
| Genotype 1a (n) | 9 | 9 |
| Median Baseline HCV RNA IU/mL | 6.9 log₁₀ | 6.7log₁₀ |
| Median HCV RNA Decline Week 2 IU/mL | -5.1 log₁₀ | -5.3 log₁₀ |
| RVR* n(%) | 7 (63.6%) | 6(60%) |
| eRVR* n(%) | 4 (36.4%) | 6 (60%) |
| cEVR* n(%) | 5 (45.5%) | 9 (90%)** |

| | | |
|---|---|---|
| RVR (rapid virologic response) = undetectable HCV RNA at Week 4 of treatment eRVR (extended rapid virologic response) = undetectable HCV RNA at both Week 4 and Week 12 of treatment cRVR (complete rapid virologic response) = undetectable HCV RNA at Week 12 of treatment *Intent-to-treat analysis; breakthrough = failure ** One subject with HCV RNA <25 IU/mL at Week 12, undetectable (UD, <10 IU/mL) on retesting. | | |

As shown in Table 2, six (54.5%) subjects experienced viral breakthrough while all subjects in Group B maintained viral suppression. Viral breakthrough occurred exclusively in individuals infected with genotype la, occurring as early as Week 3 and as late as Week 12. The two genotype 1b subjects in Group A remained HCV RNA undetectable. The six subjects with breakthrough received additional pegylated interferon alpha and ribavirin. HCV RNA fell to undetectable in two subjects and to <25 IU/mL in another two subjects while the other two had >1.5 log₁₀ decreases in HCV RNA. No deaths, significant adverse events, or discontinuations due to adverse events were recorded during the analysis period. Diarrhea was the most common adverse event and was mainly mild to moderate in severity.

The results show that while treatment with the NS5A Inhibitor of formula (I) and the NS3 Protease Inhibitor of formula (II) with or without pegylated interferon alpha and ribavirin demonstrated similar RVR rates in HCV infected genotype 1 null responders, six of the eleven subjects receiving the two small molecules alone experienced viral breakthrough by Week 12 while the four-drug combination maintained viral suppression in all subjects. Therefore, treatment with direct acting antivral agents without the pegylated interferon alpha and ribavirin would require one or more additional therapeutic agents to prevent viral breakthrough.

### Cell lines

The Huh-7 cell-line used for these studies was obtained from Dr. Ralf Bartenschlager (University of Heidelberg, Heidelberg, Germany) and was propagated in DMEM containing 10% FBS, 10 U/mL penicillin and 10 µg /mL streptomycin. The HCV replicons used in these studies were generated at Bristol-Myers Squibb using the proecedure described in WO2004014852. The coding sequence of the published genotype 1b HCV replicon (Lohmann, V., F. Korner, J.-O. Koch, U. Herian, L. Theilmann, and R. Bartenschlager 1999, Science 285:110-113) was synthesized by Operon Tehnologies, Inc. (Alameda, CA) using the sequence set forth in EMBL Accession No.AJ242652, nucleotides 1801 to 7758. The functional replicon was then assembled in plasmid pGem9zf(+) (Promega, Madison, WI) using standard molecular biology techniques. To create a replicon encoding a luciferase reporter, the gene encoding humanized Renilla Luciferase protein was introduced upstream of the neomycin phosphotransferase gene. The resulting replicon consists of (i) the HCV 5' UTR fused to the first 12 amino acids of the capsid protein, (ii) the Renilla luciferase gene, (iii) the neomycin phosphotransferase gene (neo), (iv) the IRES from encephalomyocarditis virus (EMCV), and (v) HCV NS3 to NS5B genes and the HCV 3' UTR.

HCV replicon cell lines were isolated from colonies as described by Lohman, et. al (Lohmann, V., F. Korner, J.-O. Koch, U. Herian, L. Theilmann, and R. Bartenschlager 1999, Science 285:110-113) and used for all experiments. Briefly, replicon clones were linearized with *Sca*I and RNA transcripts synthesized *in vitro* using the T7 MegaScript transcription kit (Ambion, Austin, TX) according to manufacturer's directions. Ten to 20 µg of *in vitro* transcribed replicon RNA was introduced into 4-5 X 10⁶ Huh-7 cells by transfection with DMRIE-C reagent (Invitrogen Corporation, Carlsbad, CA) following manufacturer's protocols. After 24 h, selective media containing 0.5 mg/mL Geneticin (G418, Gibco-BRL, Rockville, MD) was added and media was changed every 3 to 5 days. After approximately 4 weeks, cells were expanded for further analysis. Cells were maintained at 37 °C in DMEM (Gibco-BRL, Rockville, MD) with 10% heat inactivated calf serum (Sigma), penicillin/streptomycin, and 0.5 mg/ml G418.

### Cell culture cytotoxicity and luciferase assays

To determine compound efficacy, HCV replicon cells were plated at a density of 10⁴ per well in 96-well plates in DMEM media containing 10% FBS. Following incubation overnight, compounds serially diluted in DMSO, or DMSO alone, were added to individual wells to a final DMSO concentration of 0.5%. Cell plates were then incubated at 37°C for 3 days prior to assaying for cytotoxicity and HCV inhibition. Cell viability was measured using an Alamar Blue assay and CC₅₀ values were calculated using the median effect equation.

Plates were then washed two times with PBS and renilla luciferase activity assayed using a Dual-Glo Luciferase Assay System (Promega Corporation, Madison, WI) according to the manufacturer's directions. Plates were read on a TopCount NXT Microplate Scintillation and Luminescence Counter (Packard Instrument Company). The 50% effective concentration (EC₅₀) was calculated by using Excel Fit (Version 2.0, Build 30). Known x (compound concentration) and y (% relative to DMSO-only control wells) values were used to calculate the EC₅₀ with Excel Fit equation 205, represented as y = A + ((B-A)/(1+((C/x)^D))), where A and B equal the bottom and top plateaus of the curve, respectively, C equals the x value at the middle of the curve, and D equals the slope factor.

### Combination studies

For inhibitor combination studies, inhibitors of HCV NS5A, NS3 protease, and NS5B polymerase were each tested at eleven concentrations. Stock solutions, 200 times the desired final assay concentration, were prepared by 3-fold dilution in DMSO prior to addition to cells/media. The compounds were tested as monotherapies and in combinations at various concentration ratios. Cells were exposed to compounds for 3 days and the amount of HCV inhibition was then determined using the luciferase assay as described above. The potential cytotoxicities of these combined agents were also analyzed in parallel by Alamar blue staining. The degree of antagonism, additivity, or synergy was determined over a range of drug concentrations, and combination response curves were fit to assess the antiviral effects of the drug treatment combinations. The combined effect of the drugs in combination was analyzed using the method of Chou Chou T. Theoretical Basis, Experimental Design, and Computerized Simulation of Synergism and Antagonism in Drug Combination Studies. Pharmacological Reviews. 2006; 58(3):621-81.

All estimates were computed using biostatistical software SAS Proc NLIN, and a four-parameter logistic. All combination indices were tested for departure from additivity using isobologram methods. Asymptotic confidence intervals were also calculated for each of the combination indices. These intervals are used to test for departure from additivity by comparing the bounds to one - a lower bound of the interval greater than 1 indicates antagonism, an upper bound of less than 1 indicates synergism, and a value of 1 contained in the interval indicates additivity.

### Results

**Table 3: Triple Combination Studies Using NS3 Protease, NS5A and NS5B Inhibitors**

| **Expt^{a}** | **NS3 PI EC₅₀, nM** | **NS5A Inhibitor EC₅₀, nM** | **NS5B Inhbitor EC₅₀, nM** | **Combination Indices (confidence interval)** | | | **Overall Result** |
|---|---|---|---|---|---|---|---|
| | | | | **50% effective** | **75% effective** | **90% effective** | |
| 1 | 1.2 | 0.003 | 5.4 | 1.00 (0.92, 1.08) | 1.05 (0.93, 1.17) | 1.11 (0.91, 1.30) | Additivity |
| 2 | 0.705 | 0.002 | 2.4 | 0.93 (0.85, 1.01) | 1.01 (0.88, 1.14) | 1.1 (0.88, 1.31) | Additivity |
| 3 | 0.825 | 0.002 | 2.8 | 1.01 (0.94, 1.08) | 0.97 (0.88, 1.06) | 0.93 (0.79, 1.07) | Additivity |

| | | | | | | | |
|---|---|---|---|---|---|---|---|
| PI = Protease inhibitor ^{a} Compounds tested at a molar ratio of 250:1:1000, NS3 PI to NS5A inhibitor to NS5B inhibitor | | | | | | | |

Resistance to antiviral therapy is become a major issue in the management of patients with chronic viral infections. To achieve sustained viral responses, it will be critical to utilize combination therapies, especially those targeting distinct HCV viral targets.

These results demonstrate that combination treatment of replicon cells with the NS5A inhibitor of formula (I), the HCV NS3 protease inhibitor of formula (II), and the NS5B inhibitor of formula (III) yield additive antiviral effects. Importantly, no antagonistic effects or enhanced cytotoxicity were observed with any of these combinations. Therefore, these combinations are excellent candidates for combination regimens in HCV infected patients.

## Claims

1. A composition comprising a therapeutically effective amount of a compound of formula (I) 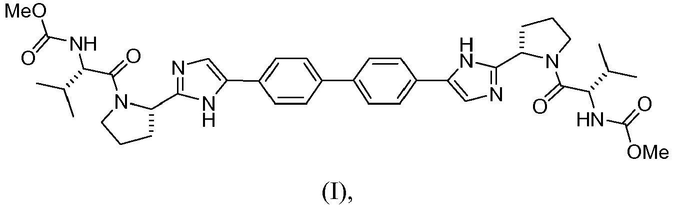 or a pharmaceutically acceptable salt thereof; a therapeutically effective amount of a compound of formula (II), 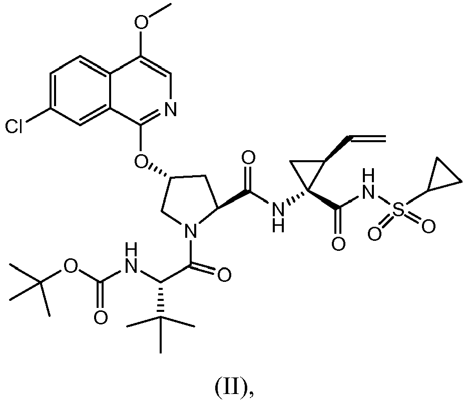 or a pharmaceutically acceptable salt thereof; a therapeutically effective amount of a compound of formula (III) 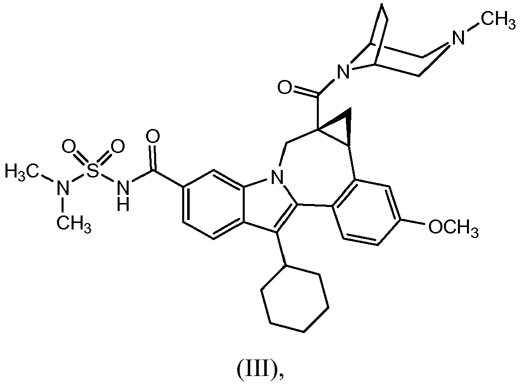 or a pharmaceutically acceptable salt thereof; and a pharmaceutically acceptable carrier.

2. The composition of claim 1 wherein the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:20:5.

3. The composition of claim 1 wherein the molar ratio of the compound of formula (I), or a pharmaceutically acceptable salt thereof, to the compound of formula (II), or a pharmaceutically acceptable salt thereof, to the compound of formula (III), or a pharmaceutically acceptable salt thereof, is 1:250:1000.

4. A composition as defined in claim 1 for use in a method of treating hepatitis C virus (HCV) infection in a patient.

## Patentansprüche

1. Zusammensetzung, umfassend eine therapeutisch wirksame Menge einer Verbindung der Formel (I) 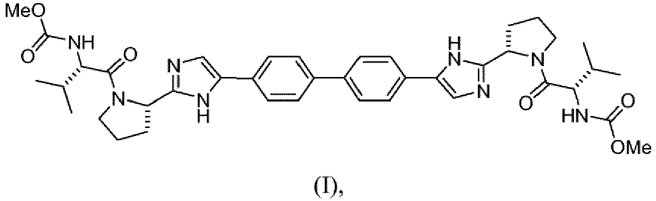 oder eines pharmazeutisch verträglichen Salzes davon; eine therapeutisch wirksame Menge einer Verbindung der Formel (II), 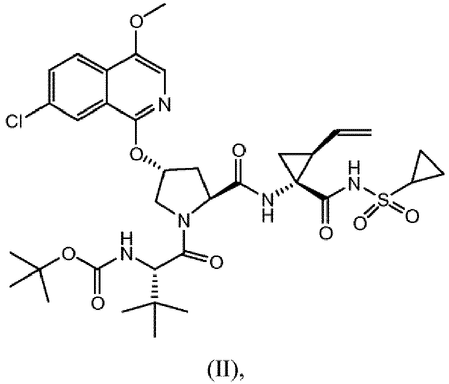 oder eines pharmazeutisch verträglichen Salzes davon; eine therapeutisch wirksame Menge einer Verbindung der Formel (III) 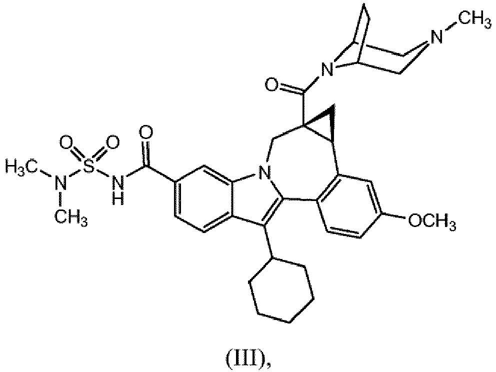 oder eines pharmazeutisch verträglichen Salzes davon; und einen pharmazeutisch verträglichen Träger.

2. Zusammensetzung nach Anspruch 1, wobei das Molverhältnis der Verbindung der Formel (I), oder eines pharmazeutisch verträglichen Salzes davon, zu der Verbindung der Formel (II), oder einem pharmazeutisch verträglichen Salz davon, zu der Verbindung der Formel (III), oder einem pharmazeutisch verträglichen Salz davon, 1:20:5 ist.

3. Zusammensetzung nach Anspruch 1, wobei das Molverhältnis der Verbindung der Formel (I), oder eines pharmazeutisch verträglichen Salzes davon, zu der Verbindung der Formel (II), oder einem pharmazeutisch verträglichen Salz davon, zu der Verbindung der Formel (III), oder einem pharmazeutisch verträglichen Salz davon, 1:250:1000 ist.

4. Zusammensetzung gemäß Definition in Anspruch 1, zur Verwendung in einem Verfahren zur Behandlung einer Hepatitis-C-Virus- (HCV-) Infektion bei einem Patienten.

## Revendications

1. Composition comprenant une quantité thérapeutiquement efficace d'un composé de la formule (I): 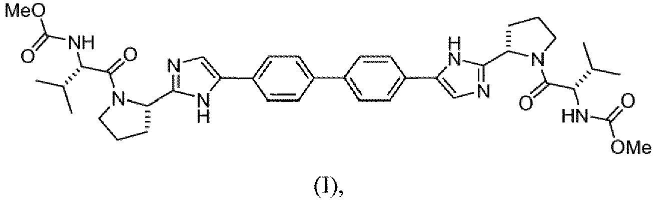 ou d'un de ses sels pharmaceutiquement acceptables; une quantité thérapeutiquement efficace d'un composé de la formule (II): 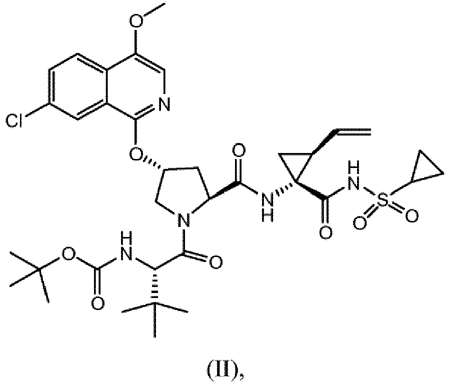 ou d'un de ses sels pharmaceutiquement acceptables; une quantité thérapeutiquement efficace d'un composé de la formule (III): 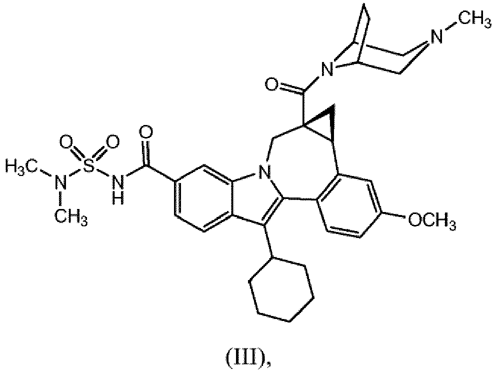 ou d'un de ses sels pharmaceutiquement acceptables; et un support pharmaceutiquement acceptable.

2. Composition selon la revendication 1, dans laquelle le rapport molaire du composé de la formule (I), ou d'un de ses sels pharmaceutiquement acceptables, sur le composé de la formule (II), ou un de ses sels pharmaceutiquement acceptables, sur le composé de la formule (III), ou un de ses sels pharmaceutiquement acceptables, est 1:20:5.

3. Composition selon la revendication 1, dans laquelle le rapport molaire du composé de la formule (I), ou d'un de ses sels pharmaceutiquement acceptables, sur le composé de la formule (II), ou un de ses sels pharmaceutiquement acceptables, sur le composé de la formule (III), ou un de ses sels pharmaceutiquement acceptables, est 1:250:1000.

4. Composition selon la revendication 1 pour une utilisation dans une méthode de traitement d'une infection par le virus de l'hépatite C (VHC) chez un patient.
